(19) Europäisches Patentamt
European Patent Office
Office européen des brevets

(11) Numéro de publication : **0 117 183**
**B1**

# (12) FASCICULE DE BREVET EUROPÉEN

(45) Date de publication du fascicule du brevet : **01.10.86**

(21) Numéro de dépôt : **84400217.0**

(22) Date de dépôt : **01.02.84**

(51) Int. Cl.⁴ : **C 07 C149/23**, C 07 C153/09, C 07 D277/46, A 61 K 31/16, A 61 K 31/265, A 61 K 31/425

(54) **Dérivés d'amides des acides mercaptoacétique et 3-mercaptopropionique pour leur utilisation dans une méthode de traitement thérapeutique du corps humain ou animal, certains dérivés nouveaux et compositions pharmaceutiques les renfermant.**

(30) Priorité : **07.02.83 FR 8301863**

(43) Date de publication de la demande : **29.08.84 Bulletin 84/35**

(45) Mention de la délivrance du brevet : **01.10.86 Bulletin 86/40**

(84) Etats contractants désignés : **AT BE CH DE FR GB IT LI LU NL SE**

(56) Documents cités :
**EP-A- 0 001 989**
**US-A- 2 709 706**
**US-A- 3 878 248**
**US-A- 4 329 495**
**CHEMICAL ABSTRACTS, vol. 95, no. 18, 2 novembre 1981, page 725, no. 161324k et Chemical Abstracts, tenth collective index, formulas, page 1291F, colonne de gauche AGRWAL MAMTA et al. : "New mercaptoacetanilides as possible analytical reagents"**

(73) Titulaire : **ROUSSEL-UCLAF**
**35, boulevard des Invalides**
**F-75007 Paris (FR)**

(72) Inventeur : **Delevallee, Françoise**
**55, rue Diderot**
**F-94320 Vincennes (FR)**
Inventeur : **Deraedt, Roger**
**23, allée J.B. Clément**
**F-93320 Pavillons-sous-Bois (FR)**
Inventeur : **Le Martret, Odile**
**42, avenue de Versailles**
**F-75016 Paris (FR)**

(74) Mandataire : **Bourgouin, André et al**
**Département des Brevets ROUSSEL UCLAF B.P. no 9**
**F-93230 Romainville (FR)**

EP 0 117 183 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des dérivés d'amides des acides mercaptoacétique et 3-mercaptopropionique définis par la formule (I) ci-après, pour leur utilisation comme médicament en thérapeutique humaine ou animale, certains dérivés nouveaux parmi ceux-ci, et les compositions pharmaceutiques les renfermant. Les produits de formule (I) sont généralement connus chimiquement, mais aucune application thérapeutique n'a été donnée à leur sujet jusqu'alors.

Le brevet EP 0 001 989 décrit des dérivés du mercaptopropanamide et notamment des dérivés portant une fonction carbonyle reliée à la fonction amide.

Ces produits sont présentés comme des immunorégulateurs les rendant utiles dans de très nombreuses maladies mais ne sont pas décrits comme analgésiques.

Par ailleurs, le brevet US 4 329 495 décrit des produits qui sont des inhibiteurs d'enképhalinase mais dont le substituant porté par la fonction amide est très différent de celui des produits de l'invention.

Certains des composés de formule (I) sont décrits comme pesticides, herbicides ou insecticides.

C'est le cas du N-(4-chorophényl) 2-mercapto acétamide décrit dans le brevet MONSANTO US 3 770 824, du 2-mercapto N-[3-(trifluorométhyl) phényl] acétamide décrit dans le brevet MONSANTO US 3 878 248, du 3-mercapto N-phényl propanamide décrit dans le brevet GOODRICH US 2 709 706. L'article d'Agra. Univ. J. Res., Sci., 1979 (Pub. 1981) 28 (3), 139-142, décrit le 2-mercapto N-(2-thiazolyl) acétamide d'un point de vue chimique.

On a découvert que les produits définis par la formule (I) indiquée ci-après et leurs sels présentaient des propriétés inhibitrices d'enképhalinase.

L'enképhalinase est une dipeptidylcarboxypeptidase qui hydrolyse de façon spécifique la méthionine et la leucine enképhaline entre le 3ème et le 4ème acide aminé, libérant ainsi un tripeptide Tyr-Gly-Gly (Swerts, J.P., Perdrisot, R., Patey, G., De La Baume, S., and Schwartz, J.C. Europ. J. Pharmacol., 1979, *57*, 279).

L'enképhalinase participe ainsi directement à la dégradation physiologique des enképhalines, ligands naturels endogènes des récepteurs opiacés. Par conséquent, les composés de formule (I), qui retardent la dégradation des enképhalines, stimulent les réactions de défense de l'organisme contre la douleur.

L'invention a donc pour objet les composés chimiques constitués par les dérivés d'amides des acides mercaptoacétique et 3-mercaptopropionique, répondant à la formule (I) :

$$R_1\ S-(CH_2)_n-\underset{\underset{O}{\|}}{C}-NH\ R_2 \qquad (I)$$

dans laquelle $R_1$ est un atome d'hydrogène ou un radical acétyle, et n est égal à 1 ou à 2, étant entendu que lorsque n est égal à 1, $R_2$ représente un radical phényle, éventuellement substitué en para par un atome de chlore ou en méta par un radical trifluorométhyle, ou un radical thiazolyle, et lorsque n est égal à 2, $R_2$ représente un radical phényle, éventuellement substitué en para par un atome de chlore, ainsi que par les sels d'addition avec les acides et les bases pharmaceutiquement acceptables desdits dérivés, pour leur utilisation comme médicament en thérapeutique humaine ou animale.

Les sels d'addition avec les acides minéraux ou organiques pharmaceutiquement acceptables peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane et éthane sulfoniques, arylsulfoniques, tels que les acides benzène et paratoluènesulfoniques et arylcarboxyliques.

Les sels de bases pharmaceutiquement acceptables peuvent être, par exemple, les sels de métaux alcalins comme le sodium et le potassium et les sels d'amines, par exemple, de triméthylamine et de diméthylamine.

Parmi les composés chimiques selon l'invention, on peut citer notamment ceux répondant à la formule (I), dont les noms suivent :

— le N-phényl 2-mercaptoacétamide,
— l'éthanethioate de S-(2-phénylamino 2-oxo éthyle),
— le N-(4-chlorophényl) 2-mercaptoacétamide,
— l'éthanethioate de S-[2-(4-chlorophénylamino) 2-oxo éthyle],
— le 2-mercapto N-[3-(trifluorométhyl) phényl] acétamide,
— le 3-mercapto N-phényl propanamide,
— l'éthanethioate de S-[3-oxo 3-(phénylamino) propyle],
— le N-(4-chlorophényl) 3-mercapto propanamide,
— l'éthanethioate de S-[3-(4-chlorophénylamino) 3-oxo propyle],
— le 2-mercapto N-(2-thiazolyl) acétamide,

— l'éthanethioate de S-[2-[(2-thiazolyl) amino] 2-oxo éthyle],

ainsi que leurs sels d'addition avec les acides et les bases pharmaceutiquement acceptables.

Parmi les composés chimiques selon l'invention, on retient plus particulièrement :

— le N-(4-chlorophényl) 2-mercapto acétamide,
— l'éthanethioate de S-[2-(4-chlorophénylamino) 2-oxo éthyle],
— le 3-mercapto N-phényl propanamide,
— le 2-mercapto N-(2-thiazolyl) acétamide,

ainsi que leurs sels d'addition avec les acides et les bases pharmaceutiquement acceptables.

Comme indiqué plus haut, les produits de formule (I) et leurs sels possèdent de très intéressantes propriétés pharmacologiques, illustrées plus loin dans la partie expérimentale, à savoir de très intéressantes propriétés inhibitrices de l'enképhalinase et présentent ainsi une très bonne activité analgésique.

En raison de ces propriétés, les composés chimiques selon la présente invention, trouvent leur emploi dans le traitement des douleurs diverses, par exemple dans le traitement des algies musculaires, articulaires ou nerveuses, dans le traitement des affections rhumatismales, des arthroses, des lumbagos, dans le traitement des douleurs dentaires, des zonas et des migraines, et aussi à titre de traitement complémentaire dans les états infectieux et fébriles.

La dose usuelle, variable selon le produit utilisé, le sujet traité et l'affection en cause, peut être par exemple de 20 mg à 2 g par jour, par voie orale, chez l'homme.

L'invention a aussi pour objet les compositions pharmaceutiques qui renferment au moins un dérivé précité, ou l'un de ses sels d'addition avec les acides ou les bases pharmaceutiquement acceptables, à titre de principe actif.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a enfin pour objet à titre de composés chimiques :

— l'éthanethioate de S-[2-(4-chlorophénylamino) 2-oxo éthyle],
— l'éthanethioate de S-[3-oxo-3-(phénylamino) propyle],
— l'éthanethioate de S-[3-(4-chlorophénylamino) 3-oxo propyle],
— l'éthanethioate de S-[2-[(2-thiazolyl) amino] 2-oxo éthyle],

ainsi que leurs sels d'addition avec les acides.

Les produits répondant à la formule générale (I) pour lesquels n = 1 peuvent être préparés comme indiqué dans J. Org. Chem., Vol. 37, N° 10, 1972, 1527.

Le shéma de préparation est le suivant :

On fait réagir sur l'acide mercapto acétique ou sur un dérivé fonctionnel de cet acide, un produit de formule $HN_2$—$R_2$, dans laquelle $R_2$ a la signification précédemment indiquée, pour obtenir un produit de formule (I) :

$$R_1\ S-CH_2-\underset{\underset{O}{\|}}{C}-NH\ R_2 \qquad (I)$$

dans laquelle $R_1$ = H et dans laquelle $R_2$ a la signification précédente.

On peut obtenir un produit de formule (I) dans laquelle

$$R_1 = CH_3-\underset{\underset{O}{\|}}{C}-,$$

en faisant réagir, par exemple, du chlorure d'acétyle sur le produit de formule (I), dans laquelle $R_1$ = H.

On peut préparer directement un produit de formule (I), dans laquelle

$$R_1 = \underset{\underset{O}{\|}}{C}-CH_3,$$

en faisant réagir sur un produit de formule $NH_2—R_2$, l'acide acétylthioacétique de formule :

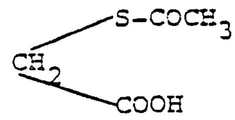

ou un dérivé fonctionnel de cet acide (produit décrit et technique indiquée dans le brevet US 2 412 700).

Les produits répondant à la formule générale (I) pour lesquels n = 2, peuvent être préparés par analogie avec le procédé de préparation indiqué dans le brevet ONDETTI US 4 053 651.

Les sels des produits de formule (I), lorsqu'ils ne sont pas connus, peuvent être préparés en faisant réagir en proportions sensiblement stœchiométriques, un acide ou une base avec lesdits produits.

Les préparations des 4 produits nouveaux cités précédemment sont données à titre d'exemple dans la partie expérimentale.

Les exemples suivants illustrent l'invention sans toutefois la limiter.

Exemple 1

Ethanethioate de S-[2-(4-chlorophénylamino) 2-oxo éthyle].

A une suspension sous azote de 2 g d'hydrure de sodium dans 19 $cm^3$ de tétrahydrofuranne, on ajoute à une température inférieure à 25 °C, une solution de 7,65 g de N-(4-chlorophényl) 2-mercapto acétamide (produit décrit et préparé selon la technique indiquée dans J. Org. Chem. Vol. 37, N° 10, 1972, 1527) dans 115 $cm^3$ de tétrahydrofuranne. Un dégagement d'hydrogène se produit. On introduit alors 3,12 g de chlorure d'acétyle, agite 1 heure à 20 °C et ajoute, sous agitation, 570 $cm^3$ d'éther éthylique et 190 $cm^3$ d'eau. Après décantation et séparation, la phase organique est lavée à l'eau, séchée et filtrée. Les solvants sont chassés sous pression réduite. On obtient 8,7 g de produit brut qui après purification par chromatographie sur silice (éluant : $CH_2Cl_2$), conduit à 4,7 g de produit attendu. F = 130 °C.

Exemple 2

Ethanethioate de S-[3-oxo-3-(phénylamino) propyle].

Stade A : Préparation du chlorure 3-(acétylthio) propionyle.

On introduit sous agitation et sous argon en 30 minutes, entre 22 et 35 °C, 100 $cm^3$ (1,4 mole) d'acide thioacétique dans 75 $cm^3$ (1,1 mole) d'acide acrylique, agite une nuit à température ambiante, puis 1 heure 30 minutes à 100 °C. Durant le retour à température ambiante, un produit cristallise. Après élimination de l'excès d'acide thioacétique sous pression réduite, on obtient 170 g d'un produit cristallisé qui est dissout dans 170 $cm^3$ d'éther éthylique. On ajoute sous argon et sous agitation, en 30 minutes, 130 $cm^3$ de chlorure de thionyle et agite une nuit à température ambiante. On élimine les solvants sous pression réduite. La distillation sous 0,5 Torr du résidu huileux obtenu, conduit à 146,9 g de produit attendu ($Eb_{0,5\ Torr}$ = 63-64 °C).

Stade B : Ethanethioate de S-[3-oxo-3-(phénylamino) propyle].

A une solution sous agitation et sous argon de 15,1 g de produit obtenu au stade A dans 500 $cm^3$ de chlorure de méthylène refroidie à — 35 °C, on ajoute en 30 minutes une solution de 16,5 $cm^3$ d'aniline dans 500 $cm^3$ de chlorure de méthylène. Après addition, on laisse revenir la température ambiante et l'agitation est maintenue pendant 16 heures. On élimine le précipité de chlorhydrate d'aniline formé par filtration. Le filtrat est lavé à l'acide chlorhydrique 0,1N puis à l'eau jusqu'à neutralité. Les eaux de lavage sont réextraites 2 fois au chlorure de méthylène. On sèche et filtre les phases organiques réunies, élimine les solvants sous pression réduite. On obtient 21,5 g de produit brut que l'on triture dans 250 $cm^3$ de pentane. On filtre, lave au pentane, sèche sous pression réduite et obtient 20 g de produit attendu. F = 99 °C.

Exemple 3

Ethanethioate de S-[3-(4-chlorophénylamino) 3-oxo propyle].

A une solution sous agitation et sous argon, de 15,7 g de produit obtenu au stade A de l'exemple 2 dans 250 $cm^3$ de chlorure de méthylène, on ajoute à — 35 °C environ en 45 minutes, une solution de 4-chloroaniline dans 250 $cm^3$ de chlorure de méthylène. On agite 22 heures et laisse revenir à température ambiante. On élimine par filtration le précipité de chlorhydrate de 4-chloroaniline formé, lave le filtrat par de l'acide chlorhydrique 0,1N puis à l'eau, sèche, élimine les solvants sous pression réduite. On dissout

les cristaux obtenus, dans du chlorure de méthylène et les traite par du charbon actif. On filtre, lave au chlorure de méthylène, élimine les solvants sous pression réduite. On obtient 24,5 g de produit brut que l'on triture dans un mélange de 100 $cm^3$ d'éther éthylique et 70 $cm^3$ de pentane. On filtre, empâte dans un mélange d'éther et pentane (6-4), filtre, sèche sous pression réduite et obtient 22 g de produit attendu. F = 103 °C.

## Exemple 4

Ethanethioate de S-[2-[(2-thiazolyl) amino] 2-oxo éthyle].

On met 10 g d'amino-thiazole en solution dans 70 $cm^3$ d'acétate d'éthyle, ajoute 20,8 $cm^3$ de triéthylamine, refroidit puis ajoute en 20 minutes entre 20 et 30 °C, 18,24 g d'éthanethioate de S-(2-chloro 2-oxo éthyle) dilué dans 20 $cm^3$ d'acétate d'éthyle (produit décrit dans CA 41 1702 et préparé selon la technique indiquée dans le brevet US 2 412 700).

On agite 2 heures à température ambiante, essore, lave à l'acétate d'éthyle et agite le solide obtenu dans 150 $cm^3$ d'eau. On filtre l'insoluble, le lave à l'eau, l'essore et le sèche sous pression réduite. On obtient 19,3 g de produit brut qui après recristallisation dans l'acétate d'éthyle, conduit à 10,4 g de produit attendu. F = 183 °C.

## Exemple 5

On a préparé des comprimés répondant à la formule :

| | |
|---|---|
| — Le N-(4-chlorophényl) 2-mercapto acétamide | 50 mg |
| — Excipient q. s. pour un comprimé terminé à | 350 mg. |

(Détail de l'excipient : lactose, talc, amidon, stéarate de magnésium).

## Exemple 6

On a préparé des comprimés répondant à la formule suivante :

| | |
|---|---|
| — Le 2-mercapto N-(2-thiazolyl) acétamide | 200 mg |
| — Excipient q. s. pour un comprimé terminé à | 350 mg. |

(Détail de l'excipient : lactose, talc, amidon, stéarate de magnésium).

## Etude biologique

1. Dosage de l'enképhalinase et détermination de l'effet des inhibiteurs.

L'activité de l'enképhalinase est déterminée dans une fraction membranaire de striatum de rat.

Les striatum sont prélevés sur glace et homogénéisés en tampon TRIS 0,05M pH 7,4 (20 fois le volume). Après une première centrifugation à 1 000 g, la fraction particulaire est obtenue à la suite de deux centrifugations de 10 minutes à 20 000 g. Le culot est ensuite réuni en suspension dans un tampon TRIS et conservé à 4 °C. Le dosage des protéines est effectué selon la méthode au bleu de Comassie.

Après une préincubation de 15 minutes à 25 °C, une aliquote de protéines est incubée 15 minutes à 25 °C en présence de 20 nanomoles de leucine-enképhaline tritiée (sur le premier acide aminé) préalablement purifiée, d'1 mM de puromycine et du produit à tester en tampon TRIS. La réaction d'hydrolyse est stoppée par addition d'acide chlorhydrique 0,2N et l'incubat subit une déprotéinisation par la chaleur (15 minutes à 95 °C). Dans ces conditions, la cinétique de la réaction est linéaire.

Les métabolites tritiés obtenus par hydrolyse sont séparés de l'enképhaline par chromatographie sur colonne de porapak Q ; ils sont élués en tampon TRIS tandis que l'enképhaline est retenue sur la colonne et recueillie ensuite en phase éthanolique.

L'activité des différents produits est exprimée en concentrations inhibitrices 50 %.

(Voir Tableau page 6)

Résultats

| Produit | Inhibition de l'enképhalinase CI 50 $10^{-5}$M |
|---|---|
| Le N-phényl 2-mercapto acétamide | 0,3 |
| Le N-(4-chlorophényl) 2-mercapto acétamide | 0,5 |
| L'éthanethioate de S-/2-(4-chloro phénylamino) 2-oxo éthyle/ | 0,6 |
| Le 3-mercapto N-phényl propanamide | 4 |
| Le 2-mercapto N-(2-thiazolyl) acétamide | 3 |
| L'éthanethioate de S-/2-/(2-thiazolyl)amino/ 2-oxo éthyle/ | 13 |

2. Test d'analgésie sur tissus inflammés. (Variante de la technique de Randall et Selitto « A method for measurement of analgesic on inflammed tissue. Arch. Int. Pharmacodyn., 1957, *111*, 409).

La technique consiste à rechercher l'effet analgésique chez le rat dont le seuil de sensibilité à la douleur a été abaissé par une inflammation.

Cette dernière est obtenue par injection de carraghénine (0,25 mg/patte) sous l'aponévrose plantaire d'une patte postérieure. La douleur est provoquée par pression mécanique appliquée sur la face dorsale de la patte et augmentée régulièrement au moyen d'un analgésimètre.

Le seuil douloureux est apprécié par la pression nécessaire pour déclencher une réaction de retrait de la patte ou une réaction de vocalisation de l'animal.

Les produits sont administrés par voie buccale 4 heures après l'injection de l'irritant et les mesures du seuil douloureux sont effectuées immédiatement avant l'injection de l'irritant puis une heure après le traitement.

Résultats

| Produit | DA en mg/kg/per os |
|---|---|
| Le N-phényl 2-mercapto acétamide | $\geq$ 20 |
| Le N-(4-chlorophényl) 2-mercapto acétamide | 4 |
| L'éthanethioate de S-/2-(4-chloro phénylamino) 2-oxo éthyle/ | 4 |
| Le 3-mercapto N-phényl propanamide | 4 |
| L'éthanethioate de S-/ 3-oxo-3-(phénylamino) propyle/ | 4 |
| Le 2-mercapto N-(2-thiazolyl) acétamide | 4 |
| L'éthanethioate de S-/2-/(2-thiazolyl) amino/ 2-oxo éthyle/ | 4 |

**Revendications**

1. Composés chimiques constitués par les dérivés d'amides des acides mercaptoacétique et 3-mercaptopropionique, répondant à la formule (I) :

$$R_1\ S-(CH_2)_n-\underset{O}{\underset{\|}{C}}-NH\ R_2 \qquad (I)$$

dans laquelle $R_1$ est un atome d'hydrogène ou un radical acétyle et n est égal à 1 ou à 2, étant entendu que lorsque n est égal à 1, $R_2$ représente un radical phényle éventuellement substitué en para par un atome de chlore ou en méta par un radical trifluorométhyle, ou un radical thiazolyle, et lorsque n est égal à 2, $R_2$ représente un radical phényle éventuellement substitué en para par un atome de chlore, ainsi que par les sels d'addition avec les acides et les bases pharmaceutiquement acceptables desdits dérivés, pour leur utilisation comme médicament en thérapeutique humaine ou animale.

2. Composés chimiques selon la revendication 1, pour leur utilisation comme inhibiteurs d'enképhalinase chez l'homme ou l'animal.

3. Composés chimiques selon la revendication 1, pour leur utilisation comme analgésiques chez l'homme ou l'animal.

4. Composés chimiques selon la revendication 1, pour leur utilisation dans le traitement des douleurs diverses et dans le traitement complémentaire des états infectieux et fébriles chez l'homme ou l'animal.

5. Composés chimiques selon la revendication 1, 2, 3 ou 4, caractérisés en ce qu'ils sont constitués par l'un quelconque des dérivés répondant à la formule (I), telle que définie à la revendication 1, dont les noms suivent :

— le N-phényl 2-mercapto acétamide,
— l'éthanethioate de S-[2-phénylamino 2-oxo éthyle],
— le N-(4-chlorophényl) 2-mercapto acétamide,
— l'éthanethioate de S-[2-(4-chlorophénylamino) 2-oxo éthyle],
— le 2-mercapto N-[3-(trifluorométhyl) phényl]acétamide,
— le 3-mercapto N-phényl propanamide,
— l'éthanethioate de S-[3-oxo 3-(phénylamino) propyle],
— le N-(4-chlorophényl) 3-mercapto propanamide,
— l'éthanethioate de S-[3-(4-chlorophénylamino) 3-oxo propyle],
— le 2-mercapto N-(2-thiazolyl) acétamide,
— l'éthanethioate de S-[2-[(2-thiazolyl) amino] 2-oxo éthyle],

ainsi que par leur sels d'addition avec les acides et les bases pharmaceutiquement acceptables.

6. Composés chimiques selon la revendication 1, 2, 3, 4 ou 5, caractérisés en ce qu'ils sont constitués par l'un quelconque des dérivés répondant à la formule (I), telle que définie à la revendication 1, dont les noms suivent :

— le N-(4-chlorophényl) 2-mercapto acétamide,
— l'éthanethioate de S-[2-(4-chlorophénylamino) 2-oxo éthyle],
— le 3-mercapto N-phényl propanamide,
— le 2-mercapto N-(2-thiazolyl) acétamide,

ainsi que par leurs sels d'addition avec les acides et les bases pharmaceutiquement acceptables.

7. Compositions pharmaceutiques, caractérisées en ce qu'elles renferment, à titre de principe actif, l'un au moins des composés chimiques définis dans l'une quelconque des revendications 1 à 6.

8. A titre de composés chimiques nouveaux :

— l'éthanethioate de S-[2-(4-chlorophénylamino) 2-oxo éthyle],
— l'éthanethioate de S-[3-oxo 3-(phénylamino) propyle],
— l'éthanethioate de S-[3-(4-chlorophénylamino) 3-oxo propyle],
— l'éthanethioate de S-[2-[(2-thiazolyl) amino] 2-oxo éthyle],

ainsi que leurs sels d'addition avec les acides.

**Claims**

1. Chemical compounds constituted by amide derivatives of mercaptoacetic acid and 3-mercaptopropionic acid, answering to the formula (I) ;

$$R_1\ S-(CH_2)_n-\underset{\underset{O}{\|}}{C}-NH\ R_2 \qquad (I)$$

in which $R_1$ is a hydrogen atom or an acetyl radical and n is a number 1 or 2, it being understood that when n is 1, $R_2$ represents a phenyl radical possibly substituted in para by a chlorine atom and in meta by a trifluoromethyl radical, or a thiazolyl radical, and when n is 2, $R_2$ represents a phenyl radical possibly substituted in para by a chlorine atom, as well as by the salts of addition with acids and pharmaceutically acceptable bases of the said derivatives for their use as a medicament in human or animal therapeutics.

2. Chemical compounds according to Claim 1, for their use as inhibitors of enkephalinase in man or animals.

3. Chemical compounds according to Claim 1, for their use as analgesics in man or animals.

4. Chemical compounds according to Claim 1, for their use in the treatment of various pains and in the complementary treatment of infectious and feverish conditions in man or animals.

5. Chemical compounds according to Claim 1, 2, 3 or 4, characterized in that they are constituted by any one of the derivatives answering to the formula (I), as defined in Claim 1, the names of which follow :

— N-phenyl-2-mercapto acetamide,
— S-[2-phenylamino-2-oxo-ethyl] ethanethioate,
— N-(4-chlorophenyl)-2-mercapto acetamide,
— S-[2-(4-chlorophenylamino)-2-oxo-ethyl] ethanethioate,
— 2-mercapto-N-[3-(trifluoromethyl)-phenyl] acetamide,
— 3-mercapto-N-phenyl propanamide,
— S-[3-oxo-3-(phenylamino) propyl] ethanethioate,
— N-(4-chlorophenyl)-3-mercapto propanamide,
— S-[3-(4-chlorophenylamino)-3-oxo-propyl] ethanethioate,
— 2-mercapto-N-(2-thiazolyl) acetamide,
— S-[2-(2-thiazolyl) amino]-2-oxo-ethyl] ethanethioate,

as well as by their salts of addition with acids and pharmaceutically acceptable bases.

6. Chemical compounds according to Claim 1, 2, 3, 4 or 5, characterized in that they are constituted by any one of the derivatives answering to the formula (I), as defined in claim 1, the names of which follow :

— N-(4-chlorophenyl)-2-mercapto acetamide,
— S-[2-(4-chlorophenylamino)-2-oxo-ethyl] ethanethioate,
— 3-mercapto-N-phenyl propanamide,
— 2-mercapto-N-(2-thiazolyl)-acetamide,

as well as by their salts of addition with acids and pharmaceutically acceptable bases.

7. Pharmaceutical compositions, characterized in that they contain, as active principle, at least one of the chemical compounds defined in any one of the Claims 1 to 6.

8. As new chemical compounds :

— S-[2-(4-chlorophenylamino)-2-oxo-ethyl] ethanethioate,
— S-[3-oxo-3-(phenylamino) propyl] ethanethioate,
— S-[3-(4-chlorophenylamino)-3-oxo-propyl] ethanethioate,
— S-[2-(2-thiazolyl) amino]-2-oxo-ethyl] ethanethioate,

as well as their salts of addition with acids.

**Patentansprüche**

1. Chemische Verbindungen, bestehend aus den Amidderivaten der Mercaptoessig- und 3-Mercaptopropionsäure der Formel (I)

$$R_1\ S-(CH_2)_n-\underset{\underset{O}{\|}}{C}-NH\ R_2 \qquad (I)$$

worin $R_1$ ein Wasserstoffatom oder einen Acetylrest darstellt und n 1 oder 2 ist, wobei, wenn n 1 ist, $R_2$ einen Phenylrest, der gegebenenfalls in para-Stellung durch ein Chloratom oder in meta-Stellung durch einen Trifluormethylrest substituiert ist, oder einen Thiazolylrest bedeutet, und wenn n 2 ist, $R_2$ einen Phenylrest bedeutet, der gegebenenfalls in para-Stellung durch ein Chloratom substituiert ist, sowie die

Additionssalze mit pharmazeutisch verträglichen Säuren und Basen dieser Derivate zu deren Verwendung als Arzneimittel in der Human- oder Veterinärtherapie.

2. Chemische Verbindungen gemäß Anspruch 1 zu deren Verwendung als Inhibitoren der Enkephalinase bei Mensch oder Tier.

3. Chemische Verbindungen gemäß Anspruch 1 zu deren Verwendung als Analgetika bei Mensch oder Tier.

4. Chemische Verbindungen gemäß Anspruch 1 zu deren Verwendung bei der Behandlung verschiedener Schmerzen und bei der ergänzenden Behandlung von infektiösen und fiebrigen Zuständen bei Mensch oder Tier.

5. Chemische Verbindungen gemäß Anspruch 1, 2, 3 oder 4, dadurch gekennzeichnet, daß sie aus einem der Derivate der Formel (I) gemäß Anspruch 1 mit den folgenden Bezeichnungen

N-Phenyl-2-mercaptoacetamid,
S-[2-Phenylamino-2-oxoethyl]-ethanthioat,
N-(4-Chlorphenyl)-2-mercaptoacetamid,
S-[2-(4-Chlorphenylamino)-2-oxoethyl]-ethanthioat,
2-Mercapto-N-[3-(trifluormethyl)-phenyl]-acetamid,
3-Mercapto-N-phenylpropanamid,
S-[3-Oxo-3-(phenylamino)-propyl]-ethanthioat,
N-(4-Chlorphenyl)-3-mercaptopropanamid,
S-[3-(4-Chlorphenylamino)-3-oxopropyl]-ethanthioat,
2-Mercapto-N-(2-thiazolyl)-acetamid,
S-[2-[(2-Thiazolyl)-amino]-2-oxoethyl]-ethanthioat

sowie aus deren pharmazeutisch verträglichen Additionssalzen mit Säuren und Basen bestehen.

6. Chemische Verbindungen gemäß Anspruch 1, 2, 3, 4 oder 5, dadurch gekennzeichnet, daß sie aus einem der Derivate der Formel (I) gemäß Anspruch 1 mit den folgenden Bezeichnungen

N-(4-Chlorphenyl)-2-mercaptoacetamid,
S-[2-(4-Chlorphenylamino)-2-oxoethyl]-ethanthioat,
3-Mercapto-N-phenylpropanamid,
2-Mercapto-N-(2-thiazolyl)-acetamid,

sowie aus deren pharmazeutisch verträglichen Additionssalzen mit Säuren und Basen bestehen.

7. Pharmazeutische Zusammensetzungen, dadurch gekennzeichnet, daß sie als Wirkstoff zumindest eine der in einem der Ansprüche 1 bis 6 definierten, chemischen Verbindungen enthalten.

8. Als neue chemische Verbindungen

S-[2-(4-Chlorphenylamino)-2-oxoethyl]-ethanthioat,
S-[3-Oxo-3-(phenylamino)-propyl]-ethanthioat,
S-[3-(4-Chlorphenylamino)-3-oxopropyl]-ethanthioat,
S-[2-[(2-Thiazolyl)-amino]-2-oxoethyl]-ethanthioat

sowie deren Additionssalze mit Säuren.